(19) Europäisches Patentamt
European Patent Office
Office européen des brevets

(11) EP 2 687 266 A1

(12) EUROPÄISCHE PATENTANMELDUNG

(43) Veröffentlichungstag:
**22.01.2014 Patentblatt 2014/04**

(51) Int Cl.:
***A61Q 5/10** (2006.01)* ***A61K 8/81** (2006.01)*

(21) Anmeldenummer: **12187854.0**

(22) Anmeldetag: **09.10.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **04.11.2011 DE 102011085753**

(71) Anmelder: **Henkel AG & Co. KGaA**
**40589 Düsseldorf (DE)**

(72) Erfinder:
- **Schettiger, Norbert**
**40723 Hilden (DE)**
- **Janßen, Frank**
**51103 Köln (DE)**

(54) **Farbintensivierung durch Polyacrylat**

(57) Mittel zum Färben und/oder Aufhellen von keratinischen Fasern, insbesondere menschlichen Haaren, enthaltend in einem kosmetischen Träger

(a) mindestens ein Acrylsäure-Copolymer enthaltend mindestens eine Struktureinheit der allgemeinen Formel (I), mindestens eine Struktureinheit der allgemeinen Formel (II) und mindestens eine Struktureinheit der allgemeinen Formel (III),

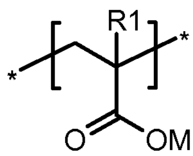

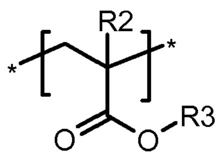

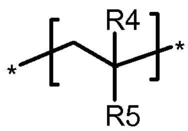

(II) (III) (I)

worin
R1, R2, R4 unabhängig voneinander für ein Wasserstoffatom oder eine $C_1$-$C_6$-Alkylgruppe stehen,
R3 für eine $C_1$-$C_{20}$-Alkylgruppe, eine $C_2$-$C_6$-Alkenylgruppe, eine $C_2$-$C_6$-Hydroxyalkylgruppe oder eine $C_2$-$C_6$-Polyhydroxyalkylgruppe steht,
R5 für ein Wasserstoffatom, eine $C_1$-$C_6$-Alkylgruppe oder eine Phenylgruppe steht,
M für ein Wasserstoffatom oder für Natrium, Kalium, ½ Magnesium oder ½ Calcium steht, und

(b) mindestens eine farbverändernde Verbindung.

EP 2 687 266 A1

**Beschreibung**

Die vorliegende Erfindung betrifft Mittel zum Färben und/oder Aufhellen von keratinischen Fasern, insbesondere menschlichen Haaren, welche spezielle Acrylsäure-Copolymere enthalten. Weiterhin betrifft die Erfindung die Verwendung dieser Mittel zur Verbesserung des Farbaufzugs bei der Färbung von keratinischen Fasern, deren Verwendung zur Verbesserung der Waschstabilität von gefärbten keratinischen Fasern sowie ein entsprechendes Verfahren.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Neben den Blondiermitteln, die eine oxidative Aufhellung der Haare durch Abbau der natürlichen Haarfarbstoffe bewirken, sind im Bereich der Haarfärbung im wesentlichen drei Typen von Haarfärbemitteln von Bedeutung: Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt. Schließlich hat in jüngster Zeit ein neuartiges Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. In solchen Verfahren wird beispielsweise 5,6-Dihydroxyindolin als Farbstoffvorprodukt eingesetzt. Bei, insbesondere mehrfacher, Anwen-dung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muß. Bei Personen mit ursprünglich mittel-blondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Nach dem Färbeprozeß ist das Haar über einen langen Zeitraum den verschiedensten Umweltbelastungen ausgesetzt. Diese reichen von der täglichen Bewitterung der Haare, beispielsweise durch Sonnenstrahlen und Haarwäschen, über mechanische, durch das Frisieren hervorgerufene Beanspruchungen bis zu chemischen Einflüssen, sofern der Konsument die Haare einem nachfolgenden Haarfärbe- oder Formungsprozess unterwirft. Die Umwelteinflüsse haben nicht nur auf die Haarstruktur selbst, sondern auch auf die nach dem Färbeprozess im Haar befindlichen Farbstoffe große Auswirkungen. Die Farbstoffe können durch die Belichtung ausbleichen, oder durch Schweiß oder Shampoonieren aus dem Haar heraus gewaschen werden. Diese Eigenschaften werden als die Echtheitseigenschaften der Farbstoffe bezeichnet. Weisen die im Verlauf der Farbausbildung gebildeten bzw. direkt eingesetzten Farbstoffe deutlich unterschiedliche Echtheiten (z. B. UV-Stabilität, Schweißechtheit, Waschechtheit etc.) auf, so kann es mit der Zeit zu einer erkennbaren und daher unerwünschten Farbverschiebung kommen. Sowohl das Auftreten von Farbverschiebungen als auch insbesondere das durch Auswaschen bedingte Verblassen der Färbungen ist vom Verbraucher unerwünscht.

Es gibt verschiedene Möglichkeiten, den durch wiederholtes Waschen bedingten Intensitätsverlust von Haarfärbungen zu reduzieren. Durch Anstreben eines von Anfang an möglichst intensiven Färbeergebnisses lässt sich der vom Verbraucher wahrgenommene Intensitätsabfall hinauszögern. Eine zweite Möglichkeit ist die Verbesserung des Farberhalts durch den Einsatz von Wirkstoffen, welche den Auswaschvorgang der Farbstoffe inhibieren bzw. reduzieren. Von besonderem Vorteil ist das Auffinden eines Wirkstoffes bzw. einer Wirkstoffkombination, welche sowohl das Farbaufzugsvermögen der Farbstoffe während des Färbeprozesses verbessert als auch deren späteres Auswaschen beim nachfolgenden Shampoonieren minimiert.

Die Aufgabe der vorliegenden Erfindung ist daher die Bereitstellung von Mitteln zum Färben und/oder Aufhellen von keratinischen Fasern, welche den Farbaufzug während der Färbung verbessern und die Penetration von Farbstoffen bzw. Farbstoffvorprodukten in die keratinischen Fasern verstärken. Weiterhin soll ein Auswaschen der Farbstoffe im Zuge wiederholter Shampoonierungen verhindert oder minimiert werden. Die erfindungsgemäßen Mittel sollen Farbintensität und Waschechtheit verbessern, ohne hierbei die Nachteile zu besitzen, die den aus dem Stand der Technik

bereits bekannten Färbemitteln zu eigen sind.

Darüber hinaus sollen mit diesen Mitteln auch Färbungen erzielt werden, die im Hinblick auf ihre weiteren Echtheitseigenschaften, wie beispielsweise ihre Licht-, Reib-, Schweiß- und Kaltwellechtheit, ein vorteilhaftes anwendungstechnisches Profil aufweisen. Schließlich ist besonders wünschenswert, Färbemittel mit gutem Egalisiervermögen bereitzustellen.

Im Kosmetikmarkt kommt der Optimierung des Farberhalts eine große Bedeutung zu, verschiedene Farbschutz-Shampoos oder Conditioner exisiteren bereits im Markt. Aus dem Stand der Technik sind diverse Substanzen bekannt, die zum Farbschutz von bereits coloriertem Haar eingesetzt werden. Insbesondere Polymere finden mit dieser Zielsetzung Anwendung.

"Silicones Used in Permanent and Semi-Permanent Hair Dyes to Reduce the Fading and Color Change Process of Dyed Hair Occuring by Wash-Out or UV Radiation", J. Cosmetic Sci., (2004) 55 (Supplement), S. 123-131, beschreibt den Einsatz von Silikonen zur Verbesserung des Farbrückhaltes. US 7 066 966 und US 7 147 672 offenbaren eine oxidative Färbezusammensetzung mit guten Waschechtheiten enthaltend ein kationisches Polyvinyllactam. US 2011/0219552 A1 offenbart eine Methode zum Schutz von gefärbtem Haar gegenüber dem Auswaschen durch Anwendung von hydrophobierten kationischen Polymeren. Schließlich betrifft die US 2011/0044924 Mittel und Verfahren zur Erhöhung der Farbintensität und zum Farbschutz von gefärbten Haaren, welche quartäre Ammoniumsalze enthalten.

Der Einsatz dieser aus dem Stand der Technik bekannten Polymere ist jedoch oftmals auch mit Nachteilen verbunden. In überraschender Weise hat sich nun gezeigt, dass Haarbehandlungsmittel enthaltend spezielle Acrylsäure-Copolymere die oben beschriebene Aufgabenstellung in optimaler Weise erfüllen, ohne mit den aus dem Stand der Technik bekannten Nachteilen behaftet zu sein. Ein erster Gegenstand der vorliegenden Erfindung sind daher Mittel zum Färben und/oder Aufhellen von keratinischen Fasern, insbesondere menschlichen Haaren, enthaltend in einem kosmetischen Träger

(a) mindestens ein Acrylsäure-Copolymer enthaltend mindestens eine Struktureinheit der allgemeinen Formel (I), mindestens eine Struktureinheit der allgemeinen Formel (II) und mindestens eine Struktureinheit der allgemeinen Formel (III),

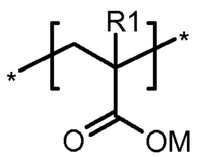
(I)

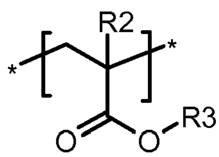
(II)

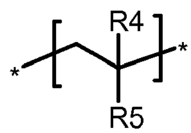
(III)

worin

| | |
|---|---|
| R1, R2, R4 | unabhängig voneinander für ein Wasserstoffatom oder eine $C_1$-$C_6$-Alkylgruppe stehen, |
| R3 | für eine $C_1$-$C_{20}$-Alkylgruppe, eine $C_2$-$C_6$-Alkenylgruppe, eine $C_2$-$C_6$-Hydroxyalkylgruppe oder eine $C_2$-$C_6$-Polyhydroxyalkylgruppe steht, |
| R5 | für ein Wasserstoffatom, eine $C_1$-$C_6$-Alkylgruppe oder eine Phenylgruppe steht, |
| M | für ein Wasserstoffatom oder für Natrium, Kalium, ½ Magnesium oder ½ Calcium steht, und |

(b) mindestens eine farbverändernde Verbindung.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Der erfindungsgemäß verwendete Begriff "Färben von Keratinfasern" umfasst jedwede Form der Farbveränderung der Fasern. Umfasst sind insbesondere die unter den Begriffen Tönung, Aufhellung, Blondierung, Bleiche, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen.

Als ersten wesentlichen Inhaltsstoff enthalten die erfindungsgemäßen Mittel mindestens ein Acrylsäure-Copolymer enthaltend mindestens eine Struktureinheit der allgemeinen Formel (I), mindestens eine Struktureinheit der allgemeinen Formel (II) und mindestens eine Struktureinheit der allgemeinen Formel (III).

Damit handelt es sich bei dem Acrylsäure-Copolymer im Rahmen der vorliegenden Erfindung um ein Copolymer, das durch Copolymerisation mindestens eines Acrylsäure- bzw. Methacrylsäuremonomers, mindestens eines Acrylsäureester- bzw. Methacrylsäureestermonomers und mindestens eines gegebenenfalls substituierten Ethenmonomers hergestellt werden kann.

Im Zuge der zu dieser Erfindung führenden Arbeiten hat sich herausgestellt, dass die erfindungsgemäße Aufgabenstellung insbesondere dann gut gelöst werden kann, wenn bei der Copolymerisation zum erfindungsgemäßen Acrylsäure-Copolymer verschiedene Acrylsäureester- bzw. Methacrylsäureestermonomere und verschiedene Ethenmonomere eingesetzt werden.

Das erfindungsgemäße Acrylsäure-Copolymer besitzt insbesondere dann vorteilhafte Eigenschaften, wenn bei der Copolymerisation zum erfindungsgemäßen Acrylsäure-Copolymer mindestens drei verschiedene Acrylsäureester- bzw. Methacrylsäureestermonomere und zwei verschiedene Ethenmonomere eingesetzt werden.

Demzufolge sind die Mittel zum Färben und/oder Aufhellen von keratinischen Fasern bevorzugt, die mindestens ein Acrylsäure-Copolymer enthalten, das mindestens eine Struktureinheit der allgemeinen Formel (I), mindestens drei unterschiedliche Struktureinheiten der allgemeinen Formel (II) und mindestens zwei unterschiedliche Struktureinheiten der allgemeinen Formel (III) enthält.

In einer bevorzugten Ausführungsform des ersten Erfindungsgegenstands sind die Mittel zum Färben und/oder Aufhellen von keratinischen Fasern daher dadurch gekennzeichnet, dass das im Mittel enthaltende Acrylsäure-Copolymer mindestens eine Struktureinheit der allgemeinen Formel (I), mindestens eine Struktureinheit der allgemeinen Formel (IIa), mindestens eine Struktureinheit der allgemeinen Formel (IIb), mindestens eine Struktureinheit der allgemeinen Formel (IIc), mindestens eine Struktureinheit der allgemeinen Formel (IIIa) und mindestens eine Struktureinheit der allgemeinen Formel (IIIb) enthält,

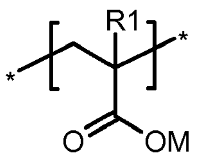
(I)

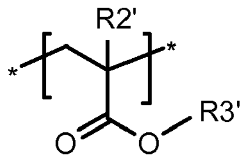
(IIa)

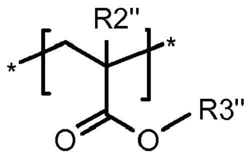
(IIb)

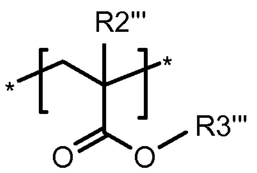
(IIc)

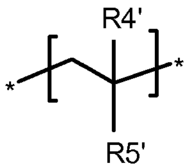
(IIIa)

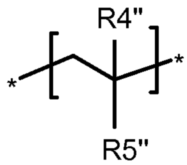
(IIIb)

worin

| | |
|---|---|
| R1, R2’, R2", R2"', R4’, R4" | unabhängig voneinander für ein Wasserstoffatom oder eine $C_1$-$C_6$-Alkylgruppe stehen, |
| R3’, R3", R3"’ | unabhängig voneinander für für eine $C_1$-$C_{20}$-Alkylgruppe, eine $C_2$-$C_6$-Alkenylgruppe, eine $C_2$-$C_6$-Hydroxyalkylgruppe oder eine $C_2$-$C_6$-Polyhydroxyalkylgruppe stehen, |
| R5’, R5" | unabhängig voneinander für ein Wasserstoffatom, eine $C_1$-$C_6$-Alkylgruppe oder eine Phenylgruppe stehen, |
| M | für ein Wasserstoffatom oder für Natrium, Kalium, ½ Magnesium oder ½ Calcium steht, |

mit der Maßgabe, dass R3’, R3" und R3"’ voneinander verschiedene Reste darstellen und
mit der Maßgabe, das R5’ und R5" voneinander verschiedene Reste darstellen.

Im Folgenden werden Beispiele für die in den Formeln (I), (II), (IIa), (IIb), (IIc), (III), (IIIa) und (IIIb) genannten Substituenten R1, R2, R2’, R2", R2"’, R3, R3’, R3", R3"’, R4, R4’, R4", R5, R5’ und R5"genannt: Beispiele für $C_1$-$C_6$-Alkylgruppen sind $-CH_3$, $-CH_2CH_3$, $-CH_2CH_2CH_3$, - $CH(CH_3)_2$, $-CH_2CH_2CH_2CH_3$, $-CH_2CH(CH_3)_2$, $-CH(CH_3)CH_2CH_3$, -C $(CH_3)_3$, $-(CH_2)_4CH_3$, $-(CH_2)_5CH_3$. Besonders bevorzugte Alkylreste sind Methyl, Ethyl und $-CH_2CH_2CH_2CH_3$- Beispiele für $C_6$-$C_{20}$ Alkylgruppen sind Hexyl, Heptyl, Octyl, Nonyl, Decyl, Dodecyl, Tetradeyl, Pentadecyl, Hexadecyl, Heptadecyl, Octadecyl, Nonadecyl und Eicosyl. Beispiele für $C_2$-$C_6$-Alkenylgruppen sind Vinyl, Prop-2-enyl (Allyl), 2-Methyl-prop-2-enyl, But-3-enyl, But-2-enyl, Pent-4-enyl oder Pent-3-enyl. Beispiele für $C_2$-$C_6$-Hydroxyalkylgruppen sind $-CH_2CH_2OH$, $-CH_2CH_2CH_2OH$, $-CH_2CH(OH)CH_3$, - $CH_2CH_2CH_2CH_2OH$, wobei die Gruppe $-CH_2CH_2OH$ bevorzugt ist. Beispiele für

$C_2$-$C_6$-Polyhydroxyalkylgruppen sind 1,2-Dihydroxyethyl, 2,3-Dihydroxypropyl, 3,4-Dihydroxybutyl und die 2,4-Dihydroxybutyl.

Bei der allgemeinen Formel (I) handelt es sich um eine Struktureinheit des erfindungsgemäßen Acrylsäure-Copolymers, die durch Einsatz eines Acrylsäure- bzw. Methacrylsäuremonomers bei der Copolymerisation erhalten wird. Bei M in Formel (I) handelt es sich bevorzugt um ein Wasserstoffatom.

Abhängig vom pH-Wert des kosmetischen Trägers kann die Säurefunktion in Formel (I) jedoch auch deprotoniert in Form einer negativ geladenen Carboxylgruppe vorliegen. In diesem Fall handelt es sich bei M um ein zur Neutralisation der Carboxygruppe vorhandenes Kation ausgewählt aus Natrium, Kalium, ½ Magnesium oder ½ Calcium. Wenn es sich bei M um Natrium oder Kalium handelt, ist dies bevorzugt.

Weiterhin ist es bevorzugt, wenn die Reste R1 und R2 der Struktureinheiten der allgemeinen Formel (I) und (II) unabhängig voneinander für eine Methylgruppe oder ein Wasserstoffatom stehen.

Der Rest R3 der Struktureinheit der allgemeinen Formel (II) steht bevorzugt für eine für eine $C_1$-$C_{20}$-Alkylgruppe.

Es ist ebenfalls bevorzugt, wenn der Rest R4 der Struktureinheit der allgemeinen Formel (III) für ein Wasserstoffatom steht und R5 für ein Wasserstoffatom oder eine Phenylgruppe steht.

Zudem ist es besonders vorteilhaft im Rahmen der vorliegenden Erfindung, wenn die Reste R1, und R2’ für eine Methylgruppe stehen und die Reste R2", R2"’, R4’, R4" und R5’ für ein Wasserstoffatom stehen.

Erfindungsgemäße Acrylsäure-Copolymere mit besonders vorteilhaften Eigenschaften werden vor allem dann erhalten, wenn die Reste R3’, R3" und R3"’ für drei verschiedene $C_1$-$C_{20}$-Alkylgruppen stehen. Insbesondere bevorzugt steht R3’ für eine Methylgruppe, R3" für eine Ethylgruppe und R3"’ für eine n-Butylgruppe (d.h. eine Gruppe $-CH_2CH_2CH_2CH_3$).

Weiterhin ist es besonders bevorzugt, wenn der Rest R5’ für ein Wasserstoffatom und der Rest R5" für eine Phenylgruppe steht.

In einer weiteren bevorzugten Ausführungsform des ersten Erfindungsgegenstands sind die Mittel zum Färben und/oder Aufhellen von keratinischen Fasern daher dadurch gekennzeichnet, dass die Reste R1, R2’ und R3’ für eine Methylgruppe stehen, die Reste R2", R2"’, R4’, R4" und R5’ für ein Wasserstoffatom stehen, R3" für eine Ethylgruppe steht, R3"’ für eine n-Butylgruppe steht und R5" für eine Phenylgruppe steht.

Bevorzugte und besonders bevorzugte Vertreter der im erfindungsgemäßen Mittel enthaltenden Acrylsäure-Copolymere sind dadurch gekennzeichnet, dass sie mindestens eine Struktureinheit der allgemeinen Formel (I), mindestens eine Struktureinheit der allgemeinen Formel (IIa), mindestens eine Struktureinheit der allgemeinen Formel (IIb), mindestens eine Struktureinheit der allgemeinen Formel (IIc), mindestens eine Struktureinheit der allgemeinen Formel (IIIa) und mindestens eine Struktureinheit der allgemeinen Formel (IIIb) enthalten, wobei die Reste R1, R2’, R2" und R2"’ unabhängig voneinander für ein Wasserstoffatom oder eine Methylgruppe stehen und die Reste R3’, R3", R3"’, R4’, R4", R5’ und R5" stehen für eine Substituentenkombination ausgewählt aus

| | R3’ | R3" | R3"’ | R4’ | R4" | R5’ | R5" |
|---|---|---|---|---|---|---|---|
| 1 | $CH_3$ | $C_2H_5$ | $C_3H_7$ | H | H | H | Phenyl |
| 2 | $CH_3$ | $C_2H_5$ | $C_4H_9$ | H | H | H | Phenyl |
| 3 | $CH_3$ | $C_2H_5$ | $C_5H_{11}$ | H | H | H | Phenyl |
| 4 | $CH_3$ | $C_2H_5$ | $C_{12}H_{25}$ | H | H | H | Phenyl |
| 5 | $CH_3$ | $C_2H_5$ | $C_{14}H_{29}$ | H | H | H | Phenyl |
| 6 | $CH_3$ | $C_2H_5$ | $C_{16}H_{33}$ | H | H | H | Phenyl |
| 7 | $CH_3$ | $C_2H_5$ | $C_{18}H_{37}$ | H | H | H | Phenyl |
| 8 | $CH_3$ | $C_3H_7$ | $C_4H_9$ | H | H | H | Phenyl |
| 9 | $CH_3$ | $C_3H_7$ | $C_5H_{11}$ | H | H | H | Phenyl |
| 10 | $CH_3$ | $C_3H_7$ | $C_{12}H_{25}$ | H | H | H | Phenyl |
| 11 | $CH_3$ | $C_3H_7$ | $C_{14}H_{29}$ | H | H | H | Phenyl |
| 12 | $CH_3$ | $C_3H_7$ | $C_{16}H_{33}$ | H | H | H | Phenyl |
| 13 | $CH_3$ | $C_3H_7$ | $C_{18}H_{37}$ | H | H | H | Phenyl |
| 14 | $CH_3$ | $C_3H_7$ | $C_3H_7$ | H | H | H | Phenyl |
| 15 | $CH_3$ | $C_4H_9$ | $C_5H_{11}$ | H | H | H | Phenyl |

(fortgesetzt)

| | R3' | R3" | R3'" | R4' | R4" | R5' | R5" |
|---|---|---|---|---|---|---|---|
| 16 | $CH_3$ | $C_4H_9$ | $C_{12}H_{25}$ | H | H | H | Phenyl |
| 17 | $CH_3$ | $C_4H_9$ | $C_{14}H_{29}$ | H | H | H | Phenyl |
| 18 | $CH_3$ | $C_4H_9$ | $C_{16}H_{33}$ | H | H | H | Phenyl |
| 19 | $CH_3$ | $C_4H_9$ | $C_{18}H_{37}$ | H | H | H | Phenyl |
| 20 | $CH_3$ | $C_5H_{11}$ | $C_{12}H_{25}$ | H | H | H | Phenyl |
| 21 | $CH_3$ | $C_5H_{11}$ | $C_{14}H_{29}$ | H | H | H | Phenyl |
| 22 | $CH_3$ | $C_5H_{11}$ | $C_{16}H_{33}$ | H | H | H | Phenyl |
| 23 | $CH_3$ | $C_5H_{11}$ | $C_{18}H_{37}$ | H | H | H | Phenyl |
| 24 | $C_2H_5$ | $C_3H_7$ | $C_4H_9$ | H | H | H | Phenyl |
| 25 | $C_2H_5$ | $C_3H_7$ | $C_5H_{11}$ | H | H | H | Phenyl |
| 26 | $C_2H_5$ | $C_3H_7$ | $C_{12}H_{25}$ | H | H | H | Phenyl |
| 27 | $C_2H_5$ | $C_3H_7$ | $C_{14}H_{29}$ | H | H | H | Phenyl |
| 28 | $C_2H_5$ | $C_3H_7$ | $C_{16}H_{33}$ | H | H | H | Phenyl |
| 29 | $C_2H_5$ | $C_3H_7$ | $C_{18}H_{37}$ | H | H | H | Phenyl |
| 30 | $C_2H_5$ | $C_4H_9$ | $C_5H_{11}$ | H | H | H | Phenyl |
| 31 | $C_2H_5$ | $C_4H_9$ | $C_{12}H_{25}$ | H | H | H | Phenyl |
| 32 | $C_2H_5$ | $C_4H_9$ | $C_{14}H_{29}$ | H | H | H | Phenyl |
| 33 | $C_2H_5$ | $C_4H_9$ | $C_{16}H_{33}$ | H | H | H | Phenyl |
| 34 | $C_3H_7$ | $C_4H_9$ | $C_5H_{11}$ | H | H | H | Phenyl |
| 35 | $C_3H_7$ | $C_4H_9$ | $C_{12}H_{25}$ | H | H | H | Phenyl |
| 36 | $C_3H_7$ | $C_4H_9$ | $C_{14}H_{29}$ | H | H | H | Phenyl |
| 37 | $C_3H_7$ | $C_4H_9$ | $C_{16}H_{33}$ | H | H | H | Phenyl |
| 38 | $C_3H_7$ | $C_4H_9$ | $C_{18}H_{37}$ | H | H | H | Phenyl |
| 39 | $C_3H_7$ | $C_4H_9$ | $C_5H_{11}$ | H | H | H | Phenyl |
| 40 | $CH_3$ | $C_2H_5$ | $C_3H_7$ | $CH_3$ | H | H | Phenyl |
| 41 | $CH_3$ | $C_2H_5$ | $C_4H_9$ | $CH_3$ | H | H | Phenyl |
| 42 | $CH_3$ | $C_2H_5$ | $C_5H_{11}$ | $CH_3$ | H | H | Phenyl |
| 43 | $CH_3$ | $C_2H_5$ | $C_{12}H_{25}$ | $CH_3$ | H | H | Phenyl |
| 44 | $CH_3$ | $C_2H_5$ | $C_{14}H_{29}$ | $CH_3$ | H | H | Phenyl |
| 45 | $CH_3$ | $C_2H_5$ | $C_{16}H_{33}$ | $CH_3$ | H | H | Phenyl |
| 46 | $CH_3$ | $C_2H_5$ | $C_{18}H_{37}$ | $CH_3$ | H | H | Phenyl |
| 47 | $CH_3$ | $C_3H_7$ | $C_4H_9$ | $CH_3$ | H | H | Phenyl |
| 48 | $CH_3$ | $C_3H_7$ | $C_5H_{11}$ | $CH_3$ | H | H | Phenyl |
| 49 | $CH_3$ | $C_3H_7$ | $C_{12}H_{25}$ | $CH_3$ | H | H | Phenyl |
| 50 | $CH_3$ | $C_3H_7$ | $C_{14}H_{29}$ | $CH_3$ | H | H | Phenyl |
| 51 | $CH_3$ | $C_3H_7$ | $C_{16}H_{33}$ | $CH_3$ | H | H | Phenyl |
| 52 | $CH_3$ | $C_3H_7$ | $C_{18}H_{37}$ | $CH_3$ | H | H | Phenyl |
| 53 | $CH_3$ | $C_3H_7$ | $C_3H_7$ | $CH_3$ | H | H | Phenyl |

(fortgesetzt)

| | R3' | R3" | R3'" | R4' | R4" | R5' | R5" |
|---|---|---|---|---|---|---|---|
| 54 | $CH_3$ | $C_4H_9$ | $C_5H_{11}$ | $CH_3$ | H | H | Phenyl |
| 55 | $CH_3$ | $C_4H_9$ | $C_{12}H_{25}$ | $CH_3$ | H | H | Phenyl |
| 56 | $CH_3$ | $C_4H_9$ | $C_{14}H_{29}$ | $CH_3$ | H | H | Phenyl |
| 57 | $CH_3$ | $C_4H_9$ | $C_{16}H_{33}$ | $CH_3$ | H | H | Phenyl |
| 58 | $CH_3$ | $C_4H_9$ | $C_{18}H_{37}$ | $CH_3$ | H | H | Phenyl |
| 59 | $CH_3$ | $C_5H_{11}$ | $C_{12}H_{25}$ | $CH_3$ | H | H | Phenyl |
| 60 | $CH_3$ | $C_5H_{11}$ | $C_{14}H_{29}$ | $CH_3$ | H | H | Phenyl |
| 61 | $CH_3$ | $C_5H_{11}$ | $C_{16}H_{33}$ | $CH_3$ | H | H | Phenyl |
| 62 | $CH_3$ | $C_5H_{11}$ | $C_{18}H_{37}$ | $CH_3$ | H | H | Phenyl |
| 63 | $C_2H_5$ | $C_3H_7$ | $C_4H_9$ | $CH_3$ | H | H | Phenyl |
| 64 | $C_2H_5$ | $C_3H_7$ | $C_5H_{11}$ | $CH_3$ | H | H | Phenyl |
| 65 | $C_2H_5$ | $C_3H_7$ | $C_{12}H_{25}$ | $CH_3$ | H | H | Phenyl |
| 66 | $C_2H_5$ | $C_3H_7$ | $C_{14}H_{29}$ | $CH_3$ | H | H | Phenyl |
| 67 | $C_2H_5$ | $C_3H_7$ | $C_{16}H_{33}$ | $CH_3$ | H | H | Phenyl |
| 68 | $C_2H_5$ | $C_3H_7$ | $C_{18}H_{37}$ | $CH_3$ | H | H | Phenyl |
| 69 | $C_2H_5$ | $C_4H_9$ | $C_5H_{11}$ | $CH_3$ | H | H | Phenyl |
| 70 | $C_2H_5$ | $C_4H_9$ | $C_{12}H_{25}$ | $CH_3$ | H | H | Phenyl |
| 71 | $C_2H_5$ | $C_4H_9$ | $C_{14}H_{29}$ | $CH_3$ | H | H | Phenyl |
| 72 | $C_2H_5$ | $C_4H_9$ | $C_{16}H_{33}$ | $CH_3$ | H | H | Phenyl |
| 73 | $C_3H_7$ | $C_4H_9$ | $C_5H_{11}$ | $CH_3$ | H | H | Phenyl |
| 74 | $C_3H_7$ | $C_4H_9$ | $C_{12}H_{25}$ | $CH_3$ | H | H | Phenyl |
| 75 | $C_3H_7$ | $C_4H_9$ | $C_{14}H_{29}$ | $CH_3$ | H | H | Phenyl |
| 76 | $C_3H_7$ | $C_4H_9$ | $C_{16}H_{33}$ | $CH_3$ | H | H | Phenyl |
| 77 | $C_3H_7$ | $C_4H_9$ | $C_{18}H_{37}$ | $CH_3$ | H | H | Phenyl |
| 78 | $C_3H_7$ | $C_4H_9$ | $C_5H_{11}$ | $CH_3$ | H | H | Phenyl |
| 79 | $CH_3$ | $C_2H_5$ | $C_3H_7$ | H | H | H | $CH_3$ |
| 80 | $CH_3$ | $C_2H_5$ | $C_4H_9$ | H | H | H | $CH_3$ |
| 81 | $CH_3$ | $C_2H_5$ | $C_5H_{11}$ | H | H | H | $CH_3$ |
| 82 | $CH_3$ | $C_2H_5$ | $C_{12}H_{25}$ | H | H | H | $CH_3$ |
| 83 | $CH_3$ | $C_2H_5$ | $C_{14}H_{29}$ | H | H | H | $CH_3$ |
| 84 | $CH_3$ | $C_2H_5$ | $C_{16}H_{33}$ | H | H | H | $CH_3$ |
| 85 | $CH_3$ | $C_2H_5$ | $C_{18}H_{37}$ | H | H | H | $CH_3$ |
| 86 | $CH_3$ | $C_3H_7$ | $C_4H_9$ | H | H | H | $CH_3$ |
| 87 | $CH_3$ | $C_3H_7$ | $C_5H_{11}$ | H | H | H | $CH_3$ |
| 88 | $CH_3$ | $C_3H_7$ | $C_{12}H_{25}$ | H | H | H | $CH_3$ |
| 89 | $CH_3$ | $C_3H_7$ | $C_{14}H_{29}$ | H | H | H | $CH_3$ |
| 90 | $CH_3$ | $C_3H_7$ | $C_{16}H_{33}$ | H | H | H | $CH_3$ |
| 91 | $CH_3$ | $C_3H_7$ | $C_{18}H_{37}$ | H | H | H | $CH_3$ |

(fortgesetzt)

| | R3’ | R3" | R3’" | R4’ | R4" | R5’ | R5" |
|---|---|---|---|---|---|---|---|
| 92 | $CH_3$ | $C_3H_7$ | $C_3H_7$ | H | H | H | $CH_3$ |
| 93 | $CH_3$ | $C_4H_9$ | $C_5H_{11}$ | H | H | H | $CH_3$ |
| 94 | $CH_3$ | $C_4H_9$ | $C_{12}H_{25}$ | H | H | H | $CH_3$ |
| 95 | $CH_3$ | $C_4H_9$ | $C_{14}H_{29}$ | H | H | H | $CH_3$ |
| 96 | $CH_3$ | $C_4H_9$ | $C_{16}H_{33}$ | H | H | H | $CH_3$ |
| 97 | $CH_3$ | $C_4H_9$ | $C_{18}H_{37}$ | H | H | H | $CH_3$ |
| 98 | $CH_3$ | $C_5H_{11}$ | $C_{12}H_{25}$ | H | H | H | $CH_3$ |
| 99 | $CH_3$ | $C_5H_{11}$ | $C_{14}H_{29}$ | H | H | H | $CH_3$ |
| 100 | $CH_3$ | $C_5H_{11}$ | $C_{16}H_{33}$ | H | H | H | $CH_3$ |
| 101 | $CH_3$ | $C_5H_{11}$ | $C_{18}H_{37}$ | H | H | H | $CH_3$ |
| 102 | $C_2H_5$ | $C_3H_7$ | $C_4H_9$ | H | H | H | $CH_3$ |
| 103 | $C_2H_5$ | $C_3H_7$ | $C_5H_{11}$ | H | H | H | $CH_3$ |
| 104 | $C_2H_5$ | $C_3H_7$ | $C_{12}H_{25}$ | H | H | H | $CH_3$ |
| 105 | $C_2H_5$ | $C_3H_7$ | $C_{14}H_{29}$ | H | H | H | $CH_3$ |
| 106 | $C_2H_5$ | $C_3H_7$ | $C_{16}H_{33}$ | H | H | H | $CH_3$ |
| 107 | $C_2H_5$ | $C_3H_7$ | $C_{18}H_{37}$ | H | H | H | $CH_3$ |
| 108 | $C_2H_5$ | $C_4H_9$ | $C_5H_{11}$ | H | H | H | $CH_3$ |
| 109 | $C_2H_5$ | $C_4H_9$ | $C_{12}H_{25}$ | H | H | H | $CH_3$ |
| 110 | $C_2H_5$ | $C_4H_9$ | $C_{14}H_{29}$ | H | H | H | $CH_3$ |
| 111 | $C_2H_5$ | $C_4H_9$ | $C_{16}H_{33}$ | H | H | H | $CH_3$ |
| 112 | $C_3H_7$ | $C_4H_9$ | $C_5H_{11}$ | H | H | H | $CH_3$ |
| 113 | $C_3H_7$ | $C_4H_9$ | $C_{12}H_{25}$ | H | H | H | $CH_3$ |
| 114 | $C_3H_7$ | $C_4H_9$ | $C_{14}H_{29}$ | H | H | H | $CH_3$ |
| 115 | $C_3H_7$ | $C_4H_9$ | $C_{16}H_{33}$ | H | H | H | $CH_3$ |
| 116 | $C_3H_7$ | $C_4H_9$ | $C_{18}H_{37}$ | H | H | H | $CH_3$ |
| 117 | $C_3H_7$ | $C_4H_9$ | $C_5H_{11}$ | H | H | H | $CH_3$ |
| 118 | $CH_3$ | $C_2H_5$ | $C_3H_7$ | $CH_3$ | H | H | $CH_3$ |
| 119 | $CH_3$ | $C_2H_5$ | $C_4H_9$ | $CH_3$ | H | H | $CH_3$ |
| 120 | $CH_3$ | $C_2H_5$ | $C_5H_{11}$ | $CH_3$ | H | H | $CH_3$ |
| 121 | $CH_3$ | $C_2H_5$ | $C_{12}H_{25}$ | $CH_3$ | H | H | $CH_3$ |
| 122 | $CH_3$ | $C_2H_5$ | $C_{14}H_{29}$ | $CH_3$ | H | H | $CH_3$ |
| 123 | $CH_3$ | $C_2H_5$ | $C_{16}H_{33}$ | $CH_3$ | H | H | $CH_3$ |
| 124 | $CH_3$ | $C_2H_5$ | $C_{18}H_{37}$ | $CH_3$ | H | H | $CH_3$ |
| 125 | $CH_3$ | $C_3H_7$ | $C_4H_9$ | $CH_3$ | H | H | $CH_3$ |
| 126 | $CH_3$ | $C_3H_7$ | $C_5H_{11}$ | $CH_3$ | H | H | $CH_3$ |
| 127 | $CH_3$ | $C_3H_7$ | $C_{12}H_{25}$ | $CH_3$ | H | H | $CH_3$ |
| 128 | $CH_3$ | $C_3H_7$ | $C_{14}H_{29}$ | $CH_3$ | H | H | $CH_3$ |
| 129 | $CH_3$ | $C_3H_7$ | $C_{16}H_{33}$ | $CH_3$ | H | H | $CH_3$ |

(fortgesetzt)

| | R3’ | R3" | R3’" | R4’ | R4" | R5’ | R5" |
|---|---|---|---|---|---|---|---|
| 130 | $CH_3$ | $C_3H_7$ | $C_{18}H_{37}$ | $CH_3$ | H | H | $CH_3$ |
| 131 | $CH_3$ | $C_3H_7$ | $C_3H_7$ | $CH_3$ | H | H | $CH_3$ |
| 132 | $CH_3$ | $C_4H_9$ | $C_5H_{11}$ | $CH_3$ | H | H | $CH_3$ |
| 133 | $CH_3$ | $C_4H_9$ | $C_{12}H_{25}$ | $CH_3$ | H | H | $CH_3$ |
| 134 | $CH_3$ | $C_4H_9$ | $C_{14}H_{29}$ | $CH_3$ | H | H | $CH_3$ |
| 135 | $CH_3$ | $C_4H_9$ | $C_{16}H_{33}$ | $CH_3$ | H | H | $CH_3$ |
| 136 | $CH_3$ | $C_4H_9$ | $C_{18}H_{37}$ | $CH_3$ | H | H | $CH_3$ |
| 137 | $CH_3$ | $C_5H_{11}$ | $C_{12}H_{25}$ | $CH_3$ | H | H | $CH_3$ |
| 138 | $CH_3$ | $C_5H_{11}$ | $C_{14}H_{29}$ | $CH_3$ | H | H | $CH_3$ |
| 139 | $CH_3$ | $C_5H_{11}$ | $C_{16}H_{33}$ | $CH_3$ | H | H | $CH_3$ |
| 140 | $CH_3$ | $C_5H_{11}$ | $C_{18}H_{37}$ | $CH_3$ | H | H | $CH_3$ |
| 141 | $C_2H_5$ | $C_3H_7$ | $C_4H_9$ | $CH_3$ | H | H | $CH_3$ |
| 142 | $C_2H_5$ | $C_3H_7$ | $C_5H_{11}$ | $CH_3$ | H | H | $CH_3$ |
| 143 | $C_2H_5$ | $C_3H_7$ | $C_{12}H_{25}$ | $CH_3$ | H | H | $CH_3$ |
| 144 | $C_2H_5$ | $C_3H_7$ | $C_{14}H_{29}$ | $CH_3$ | H | H | $CH_3$ |
| 145 | $C_2H_5$ | $C_3H_7$ | $C_{16}H_{33}$ | $CH_3$ | H | H | $CH_3$ |
| 146 | $C_2H_5$ | $C_3H_7$ | $C_{18}H_{37}$ | $CH_3$ | H | H | $CH_3$ |
| 147 | $C_2H_5$ | $C_4H_9$ | $C_5H_{11}$ | $CH_3$ | H | H | $CH_3$ |
| 148 | $C_2H_5$ | $C_4H_9$ | $C_{12}H_{25}$ | $CH_3$ | H | H | $CH_3$ |
| 149 | $C_2H_5$ | $C_4H_9$ | $C_{14}H_{29}$ | $CH_3$ | H | H | $CH_3$ |
| 150 | $C_2H_5$ | $C_4H_9$ | $C_{16}H_{33}$ | $CH_3$ | H | H | $CH_3$ |
| 151 | $C_3H_7$ | $C_4H_9$ | $C_5H_{11}$ | $CH_3$ | H | H | $CH_3$ |
| 152 | $C_3H_7$ | $C_4H_9$ | $C_{12}H_{25}$ | $CH_3$ | H | H | $CH_3$ |
| 153 | $C_3H_7$ | $C_4H_9$ | $C_{14}H_{29}$ | $CH_3$ | H | H | $CH_3$ |
| 154 | $C_3H_7$ | $C_4H_9$ | $C_{16}H_{33}$ | $CH_3$ | H | H | $CH_3$ |
| 155 | $C_3H_7$ | $C_4H_9$ | $C_{18}H_{37}$ | $CH_3$ | H | H | $CH_3$ |
| 156 | $C_3H_7$ | $C_4H_9$ | $C_5H_{11}$ | $CH_3$ | H | H | $CH_3$ |

Bei einem besonders bevorzugten Acrylsäure-Copolymer handelt es sich um das unter dem INCI-Namen bekannte Polyacrylat-15.

Eine weitere besonders bevorzugte Ausführungsform des ersten Erfindungsgegenstandes ist daher ein erfindungsgemäßes Mittel, welches dadurch gekennzeichnet ist, dass es als Acrylsäure-Copolymer Polyacrylat-15 enthält.

Die erfindungsgemäßen Mittel zum Färben und/oder Aufhellen von keratinischen Fasern enthalten das bzw. die Acrylsäure-Copolymer(e) in einer Gesamtmenge von 0,001 bis 25 Gew.-%, bevorzugt von 0,01 bis 15 Gew.-%, besonders bevorzugt von 0,1 bis Gew.-10 % und insbesondere bevorzugt von 0,5 bis 5 Gew.-% - bezogen auf das anwendungsbereite Mittel.

Eine weitere bevorzugte Ausführungsform des ersten Erfindungsgegenstandes ist daher ein Mittel zum Färben und/oder Aufhellen von keratinischen Fasern, welches dadurch gekennzeichnet ist, dass es das bzw. die Acrylsäure-Copolymer(e) (a) in einer Gesamtmenge von 0,001 bis 25 Gew.-%, bevorzugt von 0,01 bis 15 Gew.-%, besonders bevorzugt von 0,1 bis Gew.-10 % und insbesondere bevorzugt von 0,5 bis 5 Gew.-% - bezogen auf das anwendungsbereite Mittel - enthält.

Als zweiten wesentlichen Inhaltsstoff enthalten die erfindungsgemäßen Mittel mindestens eine farbverändernde

Verbindung. Unter einer farbverändernden Verbindung wird im Rahmen der vorliegenden Erfindung eine Verbindung oder eine Substanz verstanden, mittels welcher die Farbe der keratinischen Fasern verändert werden kann. Von dieser Definition mit umfasst sind sowohl Verbindungen, welche die keratinische Fasern in Richtung eines dunkleren Farbtons verändern als auch Verbindungen, nach deren Anwendung die keratinischen Fasern einen helleren als den ursprünglichen Farbton aufweisen. Unter die Definition der farbverändernden Verbindung fallen Oxidationsfarbstoffvorprodukte, direktziehende Farbstoffe, Farbstoffvorstufen naturanaloger Farbstoffe und Oxidationsmittel.

Wenn das erfindungsgemäße Mittel neben dem Acrylsäure-Copolymer als farbverändernde Verbindung mindestens ein Oxidationsfarbstoffvorprodukt und/oder mindestens einen direktziehenden Farbstoff enthält, so ist dies bevorzugt.

Eine weitere bevorzugte Ausführungsform des ersten Erfindungsgegenstandes ist daher ein erfindungsgemäßes Mittel, welches dadurch gekennzeichnet ist, dass es als farbverändernde Verbindung mindestens ein Oxidationsfarbstoffvorprodukt und/oder mindestens einen direktziehenden Farbstoff, jeweils in einer Menge von von 0,001 bis 12 Gew.-%, bevorzugt von 0,01 bis 10 Gew.-%, besonders bevorzugt von 0,1 bis 5 Gew.-% und insbesondere bevorzugt von 0,25 bis 3 Gew.-% - bezogen auf das anwendungsbereite Mittel - enthält.

Unter die Oxidationsfarbstoffvorprodukte fallen Oxidationsfarbstoffvorprodukte vom Entwickler-Typ und vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo [1,2-a]pyrazol-1-on sowie deren physiologisch verträglichen Salzen.

Besonders geeignete Oxidationsfarbstoffvorprodukte vom Kuppler-Typ sind ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methyl-amino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Im Rahmen der zu dieser Erfindung führenden Arbeiten hat es sich gezeigt, dass die erfindungsgemäße Aufgabenstellung mit Mitteln enthaltend bestimmte Entwickler/Kuppler-Kombinationen in besonderem Maße erfüllt wird. Daher sind Mittel zum Färben von keratinischen Fasern, die neben dem erfindungsgemäßen Acrylsäure-Copolymer bestimmte Kombinationen an Oxidationsfarbstoffvorprodukten enthalten, besonders bevorzugt.

Es ist bevorzugt, wenn die erfindungsgemäßen Mittel naben dem Acrylsäure-Copolymer eine der nachfolgenden Entwickler/Kuppler-Kombinationen enthalten: p-Toluylendiamin / Resorcin p-Toluylendiamin / 2-Methylresorcin; p-Toluylendiamin / 2-Amino-3-hydroxypyridin p-Toluylendiamin / 2,7-Dihydroxynaphthalin; p-Toluylendiamin / 3-Aminophenol; p-Toluylendiamin /1-Naphthol; p-Toluylendiamin / 1,5-Dihydroxynaphthalin; p-Toluylendiamin / 5-Amino-2-methylphenol; p-Toluylendiamin / 2-(2,4-Diaminophenoxy)ethanol; p-Toluylendiamin / 3-Amino-2-chlor-6-methylphenol; p-Toluylendiamin / 2,6-Dihydroxy-3,4-dimethylpyridin; p-Toluylendiamin / 3-Amino-2-methylamino-6-methoxypyridin; p-Toluylendiamin / 1,3-Bis(2,4-diaminophenyl)propan 2,4,5,6-Tetraaminopyrimidin / Resorcin; 2,4,5,6-Tetraaminopyrimidin / 2-Methylresorcin 2,4,5,6-Tetraaminopyrimidin / 2-Amino-3-hydroxypyridine; 2,4,5,6-Tetraaminopyrimidin / 2,7-Dihydroxynaphthalin; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 2-Amino-3-hydroxypyridin 4,5-Diamino-1-(2-hydroxyethyl)pyrazol / 2,7-Dihydroxynaphthalin; p-Aminophenol / Resorcin p-Aminophenol / 2-Methylresorcin; p-Aminophenol / 2-Amino-3-hydroxypyridine; p-Aminophenol / 2,7-Dihydroxynaphthalin; p-Aminophenol / 3-Aminophenol; p-Aminophenol / 1-Naphthol; p-Aminophenol / 1,5-Dihydroxynaphthalin; p-Aminophenol / 5-Amino-2-methylphenol; p-Aminophenol / 2-(2,4-Diaminophenoxy)ethanol; p-Aminophenol / 3-Amino-2-chlor-6-methylphenol; p-Aminophenol /2,6-Dihydroxy-3,4-dimethylpyridin; p-Aminophenol / 3-Amino-2-methylamino-6-methoxypyridin p-Aminophenol / 1,3-Bis(2,4-diaminophenyl)propan.

Besonders bevorzugte erfindungsgemäße Mittel enthalten neben dem Acrylsäure-Copolymer eine der nachfolgenden Kombinationen aus zwei Entwicklern und einem Kuppler: p-Toluylendiamin /2,4,5,6-Tetraaminopyrimidin / Resorcin; p-Toluylendiamin / 2,4,5,6-Tetraaminopyrimidin / 2-Methylresorcin; p-Toluylendiamin / 2,4,5,6-Tetraaminopyrimidin / 2-Amino-3-hydroxypyridin; p-Toluylendiamin / 2,4,5,6-Tetraaminopyrimidin / 2,7-Dihydroxynaphthalin.

Es wird insbesondere dann eine besonders gute Farbintensivierung bzw. ein besonders guter Farberhalt erreicht, wenn die keratinischen Fasern mit einer Formulierung gefärbt werden, die als farbverändernde Verbindungen eine Kombination aus p-Toluylendiamin, 2,4,5,6-Tetraaminopyrimidin, Resorcin, 2-Methylresorcin, 2-Amino-3-hydroxypridin und 2,7-Dihydroxynaphthalin enthält.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Im Rahmen einer weiteren bevorzugten Ausführungsform enthält das erfindungsgemäße Mittel als farbverändernde Verbindung mindestens einen direktziehenden Farbstoff. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden, die entsprechend den Anforderungen der Trägerbasis vom Fachmann ausgewählt und eingesetzt werden.

Bevorzugte anionische direktziehende Farbstoffe sind die unter den internationalen Bezeichnungen bzw. Handelsnamen Bromphenolblau, Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), Basic Blue 99, Basic Brown 16 und Basic Brown 17 sowie Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Bevorzugt ist es, wenn das erfindungsgmäße Mittel als farbverändernde Verbindung den direktziehenden Farbstoff 4-Amino-3-nitrophenol entweder allein oder in Kombination mit weiteren farbverändernden Verbindungen enthält.

In einer weiteren bevorzugten Ausführungform enthalten die erfindungsgemäßen Mittel als farbverändernde Verbindungen sowohl einen direktziehenden Farbstoff als auch ein Oxidationsfarbstoffvorprodukt vom Entwicklertyp und/oder Kupplertyp.

Es ist ebenfalls möglich, dass das erfindungsgemäße Mittel als farbverändernde Verbindung mindestens eine Farbstoffvorstufe eines naturanalogen Farbstoffes enthält. Als Farbstoffvorstufen naturanaloger Farbstoffe werden bevorzugt Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6dihydroxyindolin sowie 5,6-Dihydroxyindolin-2-carbonsäure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

Das erfindungsgemäße Mittel enthält mindestens eine farbverändernde Verbindung jeweils in einer Menge von 0,001 bis 12 Gew.-%. Handelt es sich bei dem farbverändernden Mittel um Oxidationsfarbstoffvorprodukte, direktziehende Farbstoffe und/oder Vorstufen naturanaloger Farbstoffe, so werden diese bevorzugt jeweils in einem Gewichtsanteil von 0,01 bis 10 Gew.-%, besonders bevorzugt von 0,1 bis 5 Gew.-% uns insbesondere bevorzugt von 0,25 bis 3 Gew.-% - bezogen auf das anwendungsbereite Mittel - eingesetzt.

Die erfindungsgemäßen Mittel können weiterhin als aufhellende Färbemittel oder als Aufhellmittel eingesetzt werden. Zur Erzielung des Aufhelleffekts enthalten die Mittel hierzu als farbverändernde Verbindung ein Oxidationsmittel. Bevorzugt wird als Oxidationsmittel Wasserstoffperoxid und/oder eines seiner festen Anlagerungsprodukte an organische oder anorganische Verbindungen verwendet. Beispiele für solche Anlagerungsprodukte sind an Anlagerungsprodukte an Harnstoff, Melamin sowie Natriumborat.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Handelt es sich bei dem farbverändernden Mittel um ein Oxidationsmittel, so kann dies in einer Menge von 0,001 bis 12 Gew.-% - bezogen auf das anwendungsbereite Mittel - vorhanden sein. Erfindungsgemäß bevorzugte anwendungsbereite Mittel des ersten Erfindungsgegenstands sind dadurch gekennzeichnet, dass sie 0,01 bis 10 Gew.-%, vorzugsweise 1 bis 9 Gew.-%, besonders bevorzugt 2,5 bis 8 Gew.-% und insbesondere 3 bis 6 Gew.-% Wasserstoffperoxid, jeweils bezogen auf das Gesamtgewicht des Mittels, enthalten.

Eine weitere bevorzugte Ausführungsform des ersten Erfindungsgegenstandes ist daher ein erfindungsgemäßes Mittel, welches dadurch gekennzeichnet ist, dass es als farbverändernde Verbindung mindestens ein Oxidationsmittel, ausgewählt aus Wasserstoffperoxid und seinen festen Anlagerungsprodukten an organische oder anorganische Verbindungen, in einer Menge von 0,001 bis 12 Gew.-%, bevorzugt von 0,01 bis 10 Gew.-%, vorzugsweise 1 bis 9 Gew.-%, besonders bevorzugt 2,5 bis 8 Gew.-% und insbesondere 3 bis 6 Gew.-% - bezogen auf das anwendungsbereite Mittel - enthält.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

In einer weiteren bevorzugten Ausführungsform enthalten die erfindungsgemäßen Mittel als farbverändernde Verbindungen sowohl ein Oxidationsfarbstoffvorprodukt vom Entwicklertyp und/oder Kupplertyp als auch ein Oxidationsmittel.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Wenn die erfindungsgemäßen Mittel zusätzlich Persulfate enthalten, so sind diese in einer Gesamtmenge von 0,01 bis 30 Gew.-%, bevorzugt zu 1,5 bis 28 Gew.-%, vorzugsweise 2,0 bis 25 Gew.-%, und insbesondere bevorzugt zu 5 bis 20 Gew.-% , jeweils bezogen auf das Gesamtgewicht des anwendungsbereiten Mittels, enthalten.

Das Mittel kann zur Verstärkung der Blondierwirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung kann der erfindungsgemäßen Zusammensetzung zusätzlich mindestens eine $SiO_2$-Verbindung, wie Kieselsäure oder Silicate, insbesondere Wassergläser, zugesetzt sein. Es kann erfindungsgemäß bevorzugt sein, die $SiO_2$-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der $SiO_2$-Verbindungen (ohne deren Wasseranteil) in den Mitteln wieder. Weiterhin hat es sich als vorteilhaft erweisen, wenn die Oxidationsmittelzubereitungen mindestens einen Stabilisator oder Komplexbildner enthalten. Gebräuchliche und im Rahmen der vorliegenden Erfindung bevorzugte Komplexbildner und Stabilisatoren sind beispielsweise Polyoxycarbonsäuren, Polyamine, Ethylendiamintetraessigsäure (EDTA), N-Hydroxyethylethylendiamintriessigsäure, Diethylentriaminpentaessigsäure (DTPA), Ethylendiamindibernsteinsäure (EDDS), Hydroxyethyliminodiessigsäure, Nitridodiessigsäure-3-propionsäure, Isoserindiessigsäure, N,N-Di-(2-hydroxyethyl)glycin, N-(1,2-Dicarboxy-2-hydroxyethyl)glycin, N-(1,2-Dicarboxy-2-hydroxyethyl)asparaginsäure oder Nitrilotriessigsäure (NTA), Ethylendiamindiglutarsäure (EDGA), 2-Hydroxypropylendiamindibernsteinsäure (HPDS), Glycinamid-N,N'-dibernsteinsäure (GADS), Ethylendiamin-N-N'-diglutarsäure (EDDG), 2-Hydroxypropylendiamin-N-N'-dibernsteinsäure (HPDDS), Diaminoalkyldi-(sulfobernsteinsäure) (DDS), Ethylendicysteinsäure (EDC), Ethylendiamin-N-N'-bis(ortho-hydroxyphenyl)essigsäure (EDDHA), N-2-Hydroxyethylamin-N,N-diessigsäure, Glyceryliminodiessigsäure, Iminodiessigsäure-N-2-hydroxypropylsulfonsäure, Asparaginsäure-N-carboxymethyl-N-2,5-hydroxypropyl-3-sulfonsäure,β-Alanin-N,N'-diessigsäure, Asparaginsäure-N,N'-diessigsäure, Asparaginsäure-N-monoessigsäure, Dipicolinsäure, sowie deren Salze und/oder Derivate, geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate, Aminophosphonsäuren wie Ethylendiamintetra(methylenphosphonsäure) (EDTMP), Diethylentriaminpenta(methylenphosphonsäure) (DTPMP) sowie deren höhere Homologe, oder Nitrilo-tri(methylenphosphonsäure), Phosphonopolycarbonsäuren wie 2-Phosphonobutan-1,2,4-tricarbonsäure, Cyclodextrine, sowie Alkalistannate (Natriumstannat), Alkalipyrophosphate (Tetranatriumpyrophosphat, Dinatriumpyrophosphat), Alkaliphosphate (Natriumphosphat), und Phosphorsäure sowie deren Salze.

Enthält das erfindungsgemäße Mittel als farbverändernde Verbindungen sowohl Oxidationsfarbstoffvorprodukte als auch Oxidationsmittel, so werden diese, um eine vorzeitige, unerwünschte Reaktion miteinander zu verhindern, zweckmäßigerweise getrennt voneinander konfektioniert und erst unmittelbar vor der Anwendung in Kontakt gebracht.

In einer weiteren Ausführungsform der vorliegenden Erfindung sind daher Mittel bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden, und wobei ein Container ein Mittel (A), welches in einem kosmetischen Träger mindestens ein erfindungsgemäßes Acrylsäure-Copolymer enthält, und ein weiterer Container eine Oxidationsmittelzubereitung (B), enthaltend mindestens ein Oxidationsmittel, enthält.

Die gegebenenfalls zusätzlich enthaltenen direktziehenden Farbstoffe und/oder Oxidationsfarbstoffvorprodukte vom Entwickler und/oder Kupplertyp werden in diesem Fall vorteilthafter Weise zusammen mit dem erfindungsgemäßen Acyrlsäure-Copolymer in Mittel (A) konfektioniert. In einer weiteren Ausführungsform der vorliegenden Erfindung sind Mittel bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden, und wobei ein Container ein Mittel (A), welches in einem kosmetischen Träger mindestens ein erfindungsgemäßes Acrylsäure-Copolymer enthält, und ein weiterer Container ein Mittel (B), enthaltend mindestens eine farbverändernde Verbindung, enthält.

Die erfindungsgemäßen Mittel enthalten das bzw. die Acrylsäure-Copolymer(e) und/oder die farbverändernde (n) Verbindung(en) in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die erfindungsgemäßen Mittel in eine pulverförmige oder auch tabletten-förmige Formulierung zu integrieren.

Unter wässrig-alkoholischen Lösungen sind im Sinne der vorliegenden Erfindung wässrige Lösungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die erfindungsgemäßen Zubereitungen enthalten weitere Hilfs- und Zusatzstoffe. So hat es sich erfindungsgemäß als bevorzugt erwiesen, wenn die Zubereitungen mindestens ein Verdickungsmittel enthalten. Es können sowohl organische als auch rein anorganische Verdickungsmittel zum Einsatz kommen. Gemäß einer ersten bevorzugten Ausführungsform handelt es sich bei dem Verdickungsmittel um ein anionisches, synthetisches Polymer, welches von den erfindungsgemäßen Acrylsäure-Copolymeren verschieden ist. Bevorzugte anionische Homopolymere sind unvernetzte und vernetzte Polyacrylsäuren, die beispielsweise unter dem Warenzeichnen Carbopol® im Handel erhältlich sind. Ebenfalls bevorzugt ist das Homopolymer der 2-Acrylamido-2-methylpropansulfonsäure, das beispielsweise unter der Bezeichnung Rheothik®11-80 im Handel erhältlich ist. Weitere bevorzugte anionische Copolymere sind beispielsweise Copolymere aus Acrylsäure, Methacrylsäure oder deren $C_1$-$C_6$-Alkylestern, wie sie unter der INCI-Deklaration Acrylates Copolymere vertrieben werden. Ein bevorzugtes Handelsprodukt ist beispielsweise Aculyn® 33 der Firma Rohm & Haas. Weiterhin bevorzugt sind Copolymere der Firma Rohm & Haas, die unter der Handelsbezeichnung Aculyn® 22 vertrieben werden sowie von der Firma National Starch unter den Handelsbezeichnungen Structure® 2001 und Structure® 3001. Bevorzugte anionische Copolymere sind weiterhin Sepigel®305 und Simulgel® 600 der Firma SEPPIC sowie Stabileze® QM.

In einer weiteren Ausführungsform enthalten die erfindungsgemäßen Mittel zusätzlich mindestens ein kationisches und/oder mindestens ein amphoteres Polymer.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung:

PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/ Dicaprate) und Tridecyl-polyoxypropylen-polyoxyethylen-ether (INCI-Bezeichnung: PPG-1-Trideceth-6)) im Handel erhältlich.

Ein weiteres bevorzugtes kationisches Polymer ist Salcare® SC 92.

Weitere bevorzugte kationische Polymere sind beispielsweise quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate, kationiserter Honig, beispielsweise das Handelsprodukt Honeyquat® 50, kationische Guar-Derivate, wie insbesondere die unter den Handelsnamen Cosmedia®Guar und Jaguar® vertriebenen Produkte, Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80), polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere, Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich, Vinylpyrrolidon-Vinylimidazoliummethochlorid-Copolymere, wie sie unter den Bezeichnungen Luviquat® FC 370, FC 550, FC 905 und HM 552 angeboten werden, quaternierter Polyvinylalkohol, sowie die unter den Bezeichnungen Polyquaternium 2 (z.B. Mirapol® A-15 der Firma Rhodia), Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Von besonderem Vorteil und damit ganz besonders bevorzugt ist es, wenn das erfindungsgemäße Mittel zusätzlich ein kationisches Acrylsäureester-Copolymer enthält, welches mindestens eine Struktureinheit der allgemeinen Formel (A), mindestens eine Struktureinheit der allgemeinen Formel (B) und mindestens eine Struktureinheit der allgemeinen Formel (C) enthält,

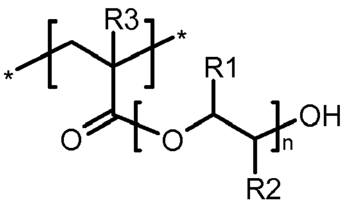

(A)

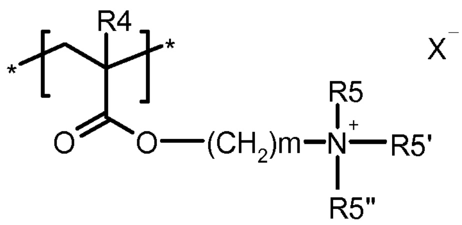

(B)

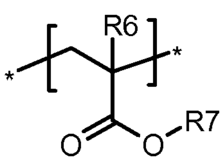

(C)

worin

| | |
|---|---|
| R1, R2, R3, R4, R6 | unabhängig voneinander für ein Wasserstoffatom oder eine $C_1$-$C_6$-Alkylgruppe stehen, |
| R5, R5', R5" | unabhängig voneinander für eine $C_1$-$C_{20}$-Alkylgruppe, eine $C_2$-$C_6$-Alkenylgruppe oder eine $C_2$-$C_6$-Hydroxyalkylgruppe stehen, |
| R7 | für eine $C_1$-$C_6$-Alkylgruppe, eine $C_2$-$C_6$-Hydroxyalkylgruppe oder eine $C_2$-$C_6$-Polyhydroxyalkylgruppe steht, |

n für eine ganze Zahl von 1 bis 50 000 steht,
m für eine ganze Zahl von 2 bis 6 steht, und
$X^-$ für ein physiologisch verträgliches Anion steht.

Bei einem besonders bevorzugten kationischen Acrylsäureester-Copolymer handelt es sich um das unter dem INCI-Namen bekannte Polyquaternium-91.

Polyquaternium-91 kann dabei in in einer Menge von 0,001 bis 20 Gew.-%, bevorzugt 0,01 bis 15 Gew.-%, besonders bevorzugt 0,1 bis 10 Gew.-% und insbesondere bevorzugt 0,5 bis 5 Gew.-% im erfindungsgemäßen Mittel enthalten sein.

Eine weitere besonders bevorzugte Ausführungsform des ersten Erfindungsgegenstandes ist daher ein Mittel zum Färben und/oder Aufhellen von keratinischen Fasern, welches dadurch gekennzeichnet ist, dass es zusätzlich - bezogen auf sein Gewicht - 0,001 bis 20 %, bevorzugt 0,01 bis 15 %, besonders bevorzugt 0,1 bis 10 % und insbesondere bevorzugt 0,5 bis 5 % Polyquaternium-91 enthält.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind.

Unter dem Begriff amphotere Polymere werden solche Polymere verstanden,

- die im Molekül sowohl freie Aminogruppen als auch freie -COOH- oder $SO_3H$-Gruppen enthalten und zur Ausbildung innerer Salze befähigt sind,
- zwitterionische Polymere, die im Molekül quartäre Ammoniumgruppen und $-COO^-$-Gruppen oder $-SO_3^-$-Gruppen enthalten, sowie
- Polymere, die -COOH-Gruppen oder $SO_3H$-Gruppen und quartäre Ammoniumgruppen enthalten.

Die in der Aufzählung genannten Polymere mit quartären Ammoniumgruppen werden erfindungsgemäß bevorzugt als amphotere Polymere eingesetzt.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Besonders bevorzugte amphotere Polymere sind Copolymere, aus mindestens einem Monomer (M1) bzw. (M2) mit dem Momomer (M3), insbesondere Copolymere aus den Monomeren (M2) und (M3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat® 280 (Nalco) vertrieben.

Erfindungsgemäß ganz besonders bevorzugt verwendete amphotere Polymere auf Basis eines Comonomers (M4) sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat® Plus 3330 (Nalco) vertrieben.

In einer weiteren bevorzugten Ausführungsform werden natürlich vorkommende Verdickungsmittel eingesetzt. Bevorzugte Verdickungsmittel dieser Ausführungsform sind beispielsweise nichtionischen Guargums. Erfindungsgemäß können sowohl modifizierte als auch unmodifizierte Guargums zum Einsatz kommen. Nichtmodifizierte Guargums werden beispielsweise unter der Handelsbezeichnung Jaguar® C von der Firma Rhone Poulenc vertrieben. Erfindungsgemäß bevorzugte modifizierte Guargums enthalten $C_1$-$C_6$-Hydroxyalkylgruppen. Bevorzugt sind die Gruppen Hydroxymethyl, Hydroxyethyl, Hydroxypropyl und Hydroxybutyl. Derart modifizierte Guargums sind im Stand der Technik bekannt und können beispielsweise durch Reaktion der Guargums mit Alkylenoxiden hergestellt werden. Der Grad der Hydroxyalkylierung, der der Anzahl der verbrauchten Alkylenoxidmoleküle im Verhältnis zur Zahl der freien Hydroxygruppen der Guargums entspricht, liegt bevorzugt zwischen 0,4 und 1,2. Derart modifizierte Guargums sind unter den Handelsbezeichnungen Jaguar® HP8, Jaguar® HP60, Jaguar® HP120, Jaguar® DC 293 und Jaguar® HP105 der Firma Rhone Poulenc im Handel erhältlich.

Gemäß dieser Ausführungsform bevorzugt sind weiterhin Biosaccharidgums mikrobiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Bevorzugte Hydroxyalkylcellulosen sind insbesondere die Hydroxyethylcellulosen, die unter den Bezeichnungen Cellosize® der Firma Amerchol und Natrosol® der Firma Hercules vertrieben werden. Geeignete Carboxyalkylcellulosen sind insbesondere die Carboxymethylcellulosen, wie sie unter den Bezeichnungen Blanose® von der Firma Aqualon, Aquasorb® und Ambergum® von der Firma Hercules und Cellgon® von der Firma Montello vertrieben werden.

Bevorzugt sind weiterhin Stärke und deren Derivate. Erfindungsgemäß einsetzbar sind natürliche, aus Pflanzen gewonnene Stärken und/oder chemisch oder physikalisch modifizierte Stärken. Besonders vorteilhaft ist eine mit einer 2-Hydroxypropylgruppe veretherte Maisstärke, wie sie beispielsweise von der Firma National Starch unter der Handelsbezeichnung Amaze® vertrieben wird. Aber auch nichtionische, vollsynthetische Polymere, wie beispielsweise Polyvinylalkohol oder Polyvinylpyrrolidinon, sind als erfindungsgemäße Verdickungsmittel einsetzbar. Bevorzugte nichtionische, vollsynthetische Polymere werden beispielsweise von der Firma BASF unter dem Handelsnamen Luviskol® vertrieben. Derartige nichtionische Polymere ermöglichen, neben ihren hervorragenden verdickenden Eigenschaften, auch eine deutliche Verbesserung des sensorischen Gefühls der resultierenden Zubereitungen.

Als anorganische Verdickungsmittel haben sich Schichtsilikate (polymere, kristalline Natriumdisilicate) als besonders geeignet im Sinne der vorliegenden Erfindung erwiesen. Insbesondere Tone, insbesondere Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate, wie beispielsweise das von der Firma Süd Chemie unter der Handelsbezeichnung Optigel® vertriebene Magnesiumschichtsilikat, sind bevorzugt. Hinsichtlich der gegebenenfalls hydratisierten $SiO_2$-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten $SiO_2$-Verbindunge können in verschiedenen Formen vorliegen. Erfindungsgemäß bevorzugt werden die $SiO_2$-Verbindunge in Form von Kieselgelen (Silicagel) oder besonders bevorzugt als Wasserglas eingesetzt. Diese $SiO_2$-Verbindunge können teilweise in wässriger Lösung vorliegen.

Erfindungsgemäß ganz besonders bevorzugt sind Wassergläser. Erfindungsgemäß besonders bevorzugte Wassergläser werden unter anderem von der Firma Henkel unter den Bezeichnungen Ferrosil® 119, Natronwasserglas 40/42, Portil® A, Portil® AW und Portil® W und von der Firma Akzo unter der Bezeichnung Britesil® C20 vertrieben.

Vorzugsweise wird dem erfindungsgemäßen Mittel weiterhin ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind. Diese Stoffe werden nachfolgend ausführlich beschrieben.

Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das $C_{12}$-$C_{18}$-Acylsarcosin.

Weiterhin hat es sich als vorteilhaft erwiesen, wenn die erfindungsgemäßen Färbe- und/oder Aufhellmittel weitere, nichtionogene grenzflächenaktive Stoffe, enthalten.

Als nichtionische Tenside eignen sich insbesondere $C_8$-$C_{22}$-Alkylmono- und oligoglycoside und deren ethoxylierte Analoga. Insbesondere die nichtethoxylierten Verbindungen haben sich als besonders geeignet erwiesen.

Besonders bevorzugt sind solche Alkylpolyglykoside der Formel RO-$(Z)_x$, bei denen R

- im Wesentlichen aus $C_8$- und $C_{10}$-Alkylgruppen,
- im Wesentlichen aus $C_{12}$- und $C_{14}$-Alkylgruppen,
- im Wesentlichen aus $C_8$- bis $C_{16}$-Alkylgruppen oder
- im Wesentlichen aus $C_{12}$- bis $C_{16}$-Alkylgruppen oder
- im Wesentlichen aus $C_{16}$ bis $C_{18}$-Alkylgruppen besteht.

Diese Verbindungen sind dadurch gekennzeichnet, dass als Zuckerbaustein Z beliebige Mono- oder Oligosaccharide eingesetzt werden können. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose,

Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar. Eine erfindungsgemäß besonders geeignete Verbindung stellt das unter der Bezeichnung Tegoamid® S 18 im Handel erhältliche Stearamidopropyl-dimethylamin dar.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis (2-Palmitoyloxyethyl)dimethylammoniumchlorid, sowie Dehyquart® F-75, Dehyquart®C-4046, Dehyquart® L80 und Dehyquart® AU-35 sind Beispiele für solche Esterquats.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

Färbe- und Aufhellprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 6 und 12, insbesondere zwischen 7 und 10,5, liegt. Bei den pH-Werten im Sinne der vorliegenden Erfindung handelt es sich um pH-Werte, die bei einer Temperatur von 22 °C gemessen wurden.

Erfindungsgemäß einsetzbare, organische Alkalisierungsmittel werden bevorzugt ausgewählt aus Alkanolaminen aus primären, sekundären oder tertiären Aminen mit einem $C_2$-$C_6$-Alkylgrundkörper, der mindestens eine Hydroxylgruppe trägt. Besonders bevorzugte Alkanolamine werden ausgewählt aus der Gruppe 2-Aminoethan-1-ol (Monoethanolamin), 2-Amino-2-methylpropan-1-ol, 2-Amino-2-methyl-propan-1,3-diol und Triethanolamin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein anorganisches Alkalisierungsmittel enthalten. Das erfindungsgemäße, anorganische Alkalisierungsmittel wird bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Ganz besonders bevorzugt sind Natriumhydroxid und/oder Kaliumhydroxid. Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin, D/L-Arginin als ein Alkalisierungsmittel im Sinne der Erfindung eingesetzt. Schließlich ist ein weiteres bevorzugtes Alkalisierungsmittel Ammoniak.

Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die zusätzlichen Wirk- und Hilfsstoffe werden in den erfindungsgemäßen Mitteln bevorzugt in Mengen von

jeweils 0,0001 bis 10 Gew.-%, insbesondere von 0,0005 bis 5 Gew.-%, bezogen auf das Gesamtgewicht der Anwendungsmischung, eingesetzt.

Ein weiterer Gegenstand der Erfindung ist die Verwendung eines erfindungsgemäßen Mittels zum Färben und/oder Aufhellen von keratinhalten Fasern, insbesondere menschlichen Haaren. Insbesondere bevorzugt ist die Verwendung eines erfindungsgemäßen Mittels zur Verbesserung des Farbaufzugs von Färbemitteln auf keratinische Fasern und/oder zur Verbesserung der Waschstabilität von gefärbten keratinischen Fasern.

Zur Anwendung der erfindungsgemäßen Mittel eignet sich insbesondere ein Verfahren zum Färben und/oder Aufhellen von keratinischen Fasern, insbesondere menschlichen Haaren, welches dadurch gekennzeichnet ist, dass ein Mittel des ersten Erfindungsgegenstands auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Färbemittel 5 bis 45 min, insbesondere 10 bis 40 min, besonders bevorzugt 15 bis 35 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die färbende und/oder aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Die erfindungsgemäßen Mittel können als Einkomponentenmittel oder als Mehrkomponentenmittel wie Zweikomponenten-Mittel oder Dreikomponenten-Mittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt. Im Fall eines oxidativen Färbeverfahrens ist ein Färbe- und Aufhellverfahren, bei dem die Aufhellcreme und das Oxidationsmittel zunächst getrennt vorliegen, bevorzugt.

Ein weiterer Gegenstand der vorliegenden Erfindung ist daher ein Verfahren zum Färben und/oder Aufhellen von keratinischen Fasern, welches dadurch gekennzeichnet ist, dass

- gewünschtenfalls ein Vorbehandlungsmittel M1 auf die Faser aufgebracht wird, dann
- ein Färbe- und/oder Aufhellmittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von 2 bis 25 Minuten wieder abgespült wird,

wobei das Mittel M2 ein erfindungsgemäßes Mittel ist.

Bezüglich weiterer bevorzugter Ausführungsformen der erfindungsgemäßen Verfahren und Verwendungen gilt mutatis mutandis das zu den erfindungsgemäßen Mitteln Gesagte.

Die folgenden Beispiele sollen den Gegenstand der vorliegenden Anmeldung näher erläutern ohne ihn jedoch in irgendeiner Form zu beschränken.

Ausführungsbeispiele

1. Intensivierung des Farbaufzugs

Es wurden die folgenden Rezepturen hergestellt. Die Mengenangaben verstehen sich, sofern nichts anderes vermerkt ist, jeweils in Gewichtsprozent. Bei V1 und V2 handelt es sich um Vergleichsrezepturen, E1 und E2 stellen die entsprechenden erfindungsgemäßen Formulierungen dar.

| Rohstoffe | V1 | E1 | V2 | E2 |
|---|---|---|---|---|
| Lanette D | 9,00 | 9,00 | 8,10 | 8,10 |
| Lorol tech. | 3,10 | 3,10 | 2,80 | 2,80 |
| Texapon NSO | 11,0 | 11,0 | 10,00 | 10,00 |

(fortgesetzt)

| Rohstoffe | V1 | E1 | V2 | E2 |
|---|---|---|---|---|
| Plantapon LGC | 5,55 | 5,55 | 5,00 | 5,00 |
| Eumulgin B1 | 0,28 | 0,28 | 0,25 | 0,25 |
| Ceteareth-20 | 0,50 | 0,50 | 0,25 | 0,25 |
| Iso-Stearinsäure | 2,22 | 2,22 | 2,00 | 2,00 |
| Myristinsäure 98-100 | 0,56 | 0,56 | 0,50 | 0,50 |
| Produkt W 37194 | 2,00 | 2,00 | 1,00 | 1,00 |
| Kaliumhydroxid 50 % | 1,63 | 1,63 | 1,20 | 1,20 |
| Syntran PC 5330 | --- | 5,00 | --- | 5,00 |
| p-Toluylendiamin, Sulfat | 1,15 | 1,15 | 1,08 | 1,08 |
| 2,4,5,6-Tetraaminopyrimidin, Sulfat | 1,10 | 1,10 | 1,02 | 1,02 |
| Resorcin | 0,14 | 0,14 | 0,11 | 0,11 |
| 2-Methylresorcin | 0,60 | 0,60 | 0,54 | 0,54 |
| 2-Amino-3-hydroxypyridin | 0,41 | 0,41 | 0,40 | 0,40 |
| 2,7-Dihydroxynaphthalin | 0,03 | 0,03 | 0,03 | 0,03 |
| 4-Amino-3-nitrophenol | 0,01 | 0,01 | --- | --- |
| 2-[(4-Amino-2-nitrophenyl)amino]benzoesäure | --- | --- | 0,09 | 0,09 |
| Natriumsulfit, wasserfrei, 96 % | 0,30 | 0,30 | 0,50 | 0,50 |
| Ascorbinsäure | 0,05 | 0,05 | 0,40 | 0,40 |
| Ammoniumsulfat techn. rein | 0,30 | 0,30 | 0,30 | 0,30 |
| Natriumsilikat 40/42 | 0,50 | 0,50 | 0,50 | 0,50 |
| 1-Hydroxyethan-1,1-diphosphonsäure 60 % | 0,20 | 0,20 | 0,20 | 0,20 |
| Ammoniak 25 % | 7,10 | 7,10 | 7,10 | 7,10 |
| Wasser | ad 100 | ad 100 | ad 100 | ad 100 |

Die Färbecremes V1 und E1 wurden unmittelbar vor der Anwendung mit der folgenden Oxidationsmittelzubereitung (Ox 1) im Verhältnis 1:1 vermischt:

Ox 1

| Rohstoff | Menge |
|---|---|
| EDTA, Dinatriumsalz | 0,15 |
| Dinatriumpyrophosphat | 0,30 |
| Natriumbenzoat | 0,04 |
| Emulgade F | 2,10 |
| Wasserstoffperoxid 50 % | 12,00 |
| Wasser | ad 100 |

Die Färbecremes V2 und E2 wurden unmittelbar vor der Anwendung mit der folgenden Oxidationsmittelzubereitung (Ox 2) im Verhältnis 1:1 vermischt:

Ox 2

| Rohstoff | Menge |
|---|---|
| Natronlauge 45 % techn. | 0,73 |
| Dipicolinsäure | 0,10 |
| Dinatriumpyrophosphat | 0,03 |
| 1-Hydroxyethan-1,1-diphosphonsäure 60 % | 1,50 |
| Texapon NSO | 2,00 |
| Dow Corning DB 110 A (nicht ionische Silikonemulsion) | 0,07 |
| Aculyn 33A | 12,00 |
| Wasserstoffperoxid 50 % | 12,00 |
| Wasser | ad 100 |

Verzeichnis der eingesetzten Rohstoffe

| | |
|---|---|
| Aculyn® 33A | ca. 28% Festkörper in Wasser; INCI-Bezeichnung: Acrylates Copolymer |
| Lanette® D | $C_{16-18}$-Fettalkohol (INCI-Bezeichnung: Cetearyl alcohol) (Cognis) |
| Lorol® tech. | $C_{12-18}$-Fettalkohol (INCI-Bezeichnung: Coconut alcohol) (Cognis) |
| Texapon® NSO | Laurylethersulfat, Natriumsalz (ca. 27,5% Aktivsubstanz; INCI-Bezeichnung: Sodium Laureth Sulfate) (Cognis) |
| Plantapon® LGC | ca. 28-34% Aktivsubstanzgehalt in Wasser; INCI-Bezeichnung: Lauryl Glucose Carboxylate, Lauryl Glucoside (Cognis) |
| Eumulgin® B 1 | Cetylstearylalkohol mit ca. 12-EO-Einheiten (INCI-Bezeichnung: Ceteareth-12) (Cognis) |
| Emulgade F | INCI-Bezeichnung: Cetearyl Alcohol, Alcohol, PEG-40 Castor Oil, Sodium Cetearylsulfate |
| Ceteareth-20 | Fettsäurealkohole, C16-C18, ethoxyliert (20 EO) |
| Produkt W 37194 | 1-Propanaminium, N,N,N-trimethyl-3-[(1-oxo-2-propenyl)amino]-, chloride, Polymer mit Natrium 2-Propenoat (INCI-Bezeichnung: Acrylamidopropyltrimoniumchloride/Acrylates Copolymer) |
| Syntran PC 5330 | Polyquaternium-91 (14 Gew.-%) und Polyacrylat-15 (4 Gew.-%) |

Vor dem Färbeprozeß wurden Haarsträhnen (Euro Naturhaar Weiß) farbmetrisch vermessen (Spectralflasch SF 450 Farbmetrik Gerät von Datacolor). Anschließend wurden die wie oben beschrieben hergestellten anwendungsbereiten Färbeformulierungen auf die Haarsträhnen gegeben und dort bei Raumtemperatur für 30 Minuten belassen. Anschließend wurden die Haarsträhnen gründlich ausgespült und im Luftstrom getrocknet. Nach dem Färben und Trocknen wurden die Haarsträhnen erneut farbmetrisch vermessen. Gemäß der nachfolgenden Formel wurde der Farbabstand (ΔE) zwischen ungefärbter und gefärbter Strähne berechnet:

$$\Delta E = \sqrt{(Lv - Ln)^2 + (av - an)^2 + (bv - bn)^2}$$

L*v*, a*v*, b*v* Farbmetrikwerte vor dem Färben
L*n*, a*n*, b*n* Farbmetrikwerte nach dem Färben

| Bestimmung des Farbaufzugsvermögens | | L | a | b | ΔE |
|---|---|---|---|---|---|
| Euronaturhaar weiß, ungefärbt | vor dem Färben | 70,50 | 2,55 | 21,11 | |
| Färbung mit V1 | nach dem Färben | 21,92 | 12,29 | 6,97 | 51,5 |
| Färbung mit E1 | nach dem Färben | 19,98 | 12,39 | 6,60 | 53,5 |
| Färbung mit V2 | nach dem Färben | 20,82 | 11,62 | 5,02 | 53,0 |
| Färbung mit E2 | nach dem Färben | 18,89 | 9,93 | 4,07 | 54,9 |

Je größer der Farbabstand zwischen ungefärbter Strähne und gefärbter Strähne ist, desto stärker ist der durch die Färbung resultierende Farbaufzug. Bei Anwendung der erfindungsgemäßen Formulierungen E1 und E2 wurde jeweils im Vergleich zur entsprechenden Vergleichsformulierung ein deutlich intensiveres Farbergebnis erhalten.

2. Verbesserung der Waschechtheit

Zur Bestimmung der Waschechtheiten bzw. des Farbrückhaltes wurden die mit den Rezepturen V2 und E2 unter Punkt 1. gefärbten Haarsträhnen in ein Ultraschallbad gegeben und nach einem standardisierten Verfahren 6, 12, 18 und 24 Haarwäschen unterzogen. Jeweils nach 6 Haarwäschen wurden die Strähnen aus dem Ultraschallbad entnommen, getrocknet und farbmetrisch vermessen.

Auf diese Weise wurden die farbmetrischen Daten direkt nach der Färbung der Haarsträhnen (0 HW) sowie nach 6, 12, 18 und 24 Haarwäschen bestimmt. Gemäß der nachfolgenden Formel wurde jeweils der Farbabstand (ΔE-Wert) zwischen der ungewaschenen und der definiert gewaschenen Strähne berechnet:

$$\Delta E = \sqrt{(L_0 - L_x)^2 + (a_0 - a_x)^2 + (b_0 - b_x)^2}$$

$L_0$, $a_0$, $b_0$ Farbmetrikwerte nach 0 Haarwäschen

$L_x$, $a_x$, $b_x$ Farbmetrikwerte jeweils nach 6, 12, 18 bzw. 24 Haarwäschen

| Bestimmung der Waschechtheiten | Haarwäschen (HW) | L | a | b | ΔE |
|---|---|---|---|---|---|
| V2 | 0 | 20,82 | 11,62 | 5,02 | |
| | 6 | 21,97 | 14,62 | 7,39 | 4,0 |
| | 12 | 21,75 | 14,35 | 7,08 | 3,5 |
| | 18 | 21,96 | 14,35 | 6,90 | 3,5 |
| | 24 | 23,40 | 15,70 | 8,96 | 6,2 |
| E2 | 0 | 18,89 | 9,93 | 4,07 | |
| | 6 | 19,43 | 11,95 | 5,50 | 2,5 |
| | 12 | 19,44 | 12,51 | 5,92 | 3,2 |
| | 18 | 19,44 | 12,65 | 6,08 | 3,4 |
| | 24 | 19,88 | 13,11 | 6,56 | 4,2 |

Je größer der Farbabstand (ΔE-Wert) zwischen ungewaschener und gewaschener Strähne ist, desto schlechter ist deren Waschechtheit. Ein Vergleich der mit den Rezepturen V2 und E2 gefärbten Strähnen zeigt, dass die mit der erfindungsgemäßen Rezeptur E2 gefärbten Strähnen bei allen Waschzyklen einen kleineren Farbabstand und damit eine bessere Waschechtheit aufweisen.

**Patentansprüche**

1. Mittel zum Färben und/oder Aufhellen von keratinischen Fasern, insbesondere menschlichen Haaren, enthaltend in einem kosmetischen Träger

(a) mindestens ein Acrylsäure-Copolymer enthaltend mindestens eine Struktureinheit der allgemeinen Formel (I), mindestens eine Struktureinheit der allgemeinen Formel (II) und mindestens eine Struktureinheit der allgemeinen Formel (III),

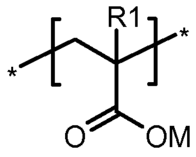
(I)

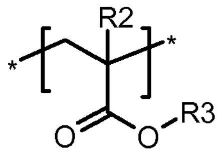
(II)

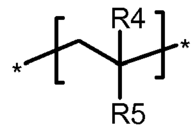
(III)

worin

R1, R2, R4 unabhängig voneinander für ein Wasserstoffatom oder eine $C_1$-$C_6$-Alkylgruppe stehen,
R3 für eine $C_1$-$C_{20}$-Alkylgruppe, eine $C_2$-$C_6$-Alkenylgruppe, eine $C_2$-$C_6$-Hydroxyalkylgruppe oder eine $C_2$-$C_6$-Polyhydroxyalkylgruppe steht,
R5 für ein Wasserstoffatom, eine $C_1$-$C_6$-Alkylgruppe oder eine Phenylgruppe steht,
M für ein Wasserstoffatom oder für Natrium, Kalium, ½ Magnesium oder ½ Calcium steht, und

(b) mindestens eine farbverändernde Verbindung.

2. Mittel nach Anspruch 1, **dadurch gekennzeichnet, dass** das im Mittel enthaltende Acrylsäure-Copolymer mindestens eine Struktureinheit der allgemeinen Formel (I), mindestens eine Struktureinheit der allgemeinen Formel (IIa), mindestens eine Struktureinheit der allgemeinen Formel (IIb), mindestens eine Struktureinheit der allgemeinen Formel (IIc), mindestens eine Struktureinheit der allgemeinen Formel (IIIa) und mindestens eine Struktureinheit der allgemeinen Formel (IIIb) enthält,

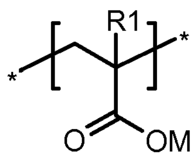
(I)

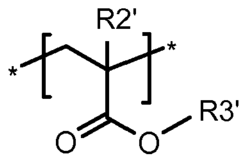
(IIa)

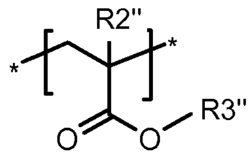
(IIb)

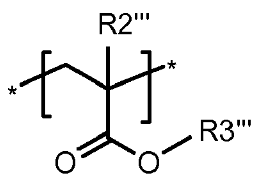
(IIc)

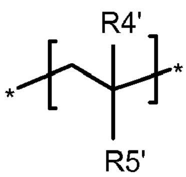
(IIIa)

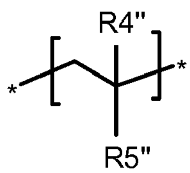
(IIIb)

worin

R1, R2', R2", R2"', R4', R4" unabhängig voneinander für ein Wasserstoffatom oder eine $C_1$-$C_6$-Alkylgruppe stehen,
R3', R3", R3"' unabhängig voneinander für für eine $C_1$-$C_{20}$-Alkylgruppe, eine $C_2$-$C_6$-Alkenylgruppe, eine $C_2$-$C_6$-Hydroxyalkylgruppe oder eine $C_2$-$C_6$-Polyhydroxyalkylgruppe stehen,
R5', R5" unabhängig voneinander für ein Wasserstoffatom, eine $C_1$-$C_6$-Alkylgruppe oder eine Phenylgruppe stehen,

M für ein Wasserstoffatom oder für Natrium, Kalium, ½ Magnesium oder ½ Calcium steht,

mit der Maßgabe, dass R3', R3" und R3"' voneinander verschiedene Reste darstellen und
mit der Maßgabe, das R5' und R5" voneinander verschiedene Reste darstellen.

3. Mittel nach Anspruch 2, **dadurch gekennzeichnet, dass** die Reste R1, R2' und R3' für eine Methylgruppe stehen, die Reste R2", R2"', R4', R4" und R5' für ein Wasserstoffatom stehen, R3" für eine Ethylgruppe steht, R3"' für eine n-Butylgruppe steht und R5" für eine Phenylgruppe steht.

4. Mittel nach einem der Ansprüche 1 bis 3, **dadurch gekennzeichnet, dass** es als Acrylsäure-Copolymer Polyacrylat-15 enthält.

5. Mittel nach einem der Ansprüche 1 bis 4, **dadurch gekennzeichnet, dass** es das bzw. die Acrylsäure-Copolymer (e) (a) in einer Gesamtmenge von 0,001 bis 25 Gew.-%, bevorzugt von 0,01 bis 15 Gew.-%, besonders bevorzugt von 0,1 bis Gew.-10 % und insbesondere bevorzugt von 0,5 bis 5 Gew.-% - bezogen auf das anwendungsbereite Mittel - enthält.

6. Mittel nach einem der Ansprüche 1 bis 5, **dadurch gekennzeichnet, dass** es als farbverändernde Verbindung mindestens ein Oxidationsfarbstoffvorprodukt und/oder mindestens einen direktziehenden Farbstoff, jeweils in einer Menge von 0,001 bis 12 Gew.-%, bevorzugt von 0,01 bis 10 Gew.-%, besonders bevorzugt von 0,1 bis 5 Gew.-% und insbesondere bevorzugt von 0,25 bis 3 Gew.-% - bezogen auf das anwendungsbereite Mittel - enthält.

7. Mittel nach einem der Ansprüche 1 bis 6, **dadurch gekennzeichnet, dass** es als farbverändernde Verbindungen eine der folgenden Entwickler / Kuppler-Kombinationen enthält: p-Toluylendiamin / Resorcin; p-Toluylendiamin / 2-Methylresorcin; p-Toluylendiamin / 2-Amino-3-hydroxypyridin; p-Toluylendiamin / 2,7-Dihydroxynaphthalin; p-Toluylendiamin / 3-Aminophenol; p-Toluylendiamin / 1-Naphthol; p-Toluylendiamin / 1,5-Dihydroxynaphthalin; p-Toluylendiamin / 5-Amino-2-methylphenol; p-Toluylendiamin / 2-(2,4-Diaminophenoxy)ethanol; p-Toluylendiamin / 3-Amino-2-chlor-6-methylphenol; p-Toluylendiamin / 2,6-Dihydroxy-3,4-dimethylpyridin; p-Toluylendiamin / 3-Amino-2-methylamino-6-methoxypyridin; p-Toluylendiamin / 1,3-Bis(2,4-diaminophenyl)propan; 2,4,5,6-Tetraaminopyrimidin / Resorcin; 2,4,5,6-Tetraaminopyrimidin / 2-Methylresorcin; 2,4,5,6-Tetraaminopyrimidin / 2-Amino-3-hydroxypyridin; 2,4,5,6-Tetraaminopyrimidin /2,7-Dihydroxynaphthalin; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 2-Amino-3-hydroxypyridin; 4,5-Diamino-1-(2-hydroxyethyl)pyrazol / 2,7-Dihydroxynaphthalin; p-Aminophenol / Resorcin; p-Aminophenol / 2-Methylresorcin; p-Aminophenol / 2-Amino-3-hydroxypyridin; p-Aminophenol / 2,7-Dihydroxynaphthalin; p-Aminophenol /3-Aminophenol; p-Aminophenol / 1-Naphthol; p-Aminophenol / 1,5-Dihydroxynaphthalin; p-Aminophenol / 5-Amino-2-methylphenol; p-Aminophenol / 2-(2,4-Diaminophenoxy)-ethanol; p-Aminophenol / 3-Amino-2-chlor-6-methylphenol; p-Aminophenol / 2,6-Dihydroxy-3,4-dimethylpyridin; p-Aminophenol / 3-Amino-2-methylamino-6-methoxypyridin; p-Aminophenol / 1,3-Bis(2,4-diaminophenyl)propan.

8. Mittel nach einem der Ansprüche 1 bis 7, **dadurch gekennzeichnet, dass** es als farbverändernde Verbindungen eine der folgenden Kombinationen aus zwei Entwicklern und einem Kuppler enthält: p-Toluylendiamin / 2,4,5,6-Tetraaminopyrimidin / Resorcin; p-Toluylendiamin / 2,4,5,6-Tetraaminopyrimidin / 2-Methylresorcin; p-Toluylendiamin /2,4,5,6-Tetraaminopyrimidin / 2-Amino-3-hydroxypyridin; p-Toluylendiamin / 2,4,5,6-Tetraaminopyrimidin / 2,7-Dihydroxynaphthalin.

9. Mittel nach einem der Ansprüche 1 bis 8, **dadurch gekennzeichnet, dass** es als farbverändernde Verbindung mindestens einen nichtionischen direktziehenden Farbstoff aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol enthält.

10. Mittel nach einem der Ansprüche 1 bis 9, **dadurch gekennzeichnet, dass** es als farbverändernde Verbindung den direktziehenden Farbstoff 4-Amino-3-nitrophenol entweder allein oder in Kombination mit weiteren farbverändernden

Verbindungen enthält.

11. Mittel nach einem der Ansprüche 1 bis 10, **dadurch gekennzeichnet, dass** es als farbverändernde Verbindungen sowohl einen direktziehenden Farbstoff als auch ein Oxidationsfarbstoffvorprodukt vom Entwicklertyp und/oder Kupplertyp enthält.

12. Mittel nach einem der Ansprüche 1 bis 11, **dadurch gekennzeichnet, dass** es als farbverändernde Verbindung mindestens ein Oxidationsmittel, ausgewählt aus Wasserstoffperoxid und seinen festen Anlagerungsprodukten an organische oder anorganische Verbindungen, in einer Menge von 0,001 bis 12 Gew.-%, bevorzugt von 0,01 bis 10 Gew.-%, vorzugsweise 1 bis 9 Gew.-%, besonders bevorzugt 2,5 bis 8 Gew.-% und insbesondere 3 bis 6 Gew.-% - bezogen auf das anwendungsbereite Mittel - enthält.

13. Mittel nach einem der Ansprüche 1 bis 12, **dadurch gekennzeichnet, dass** es zusätzlich - bezogen auf sein Gewicht - 0,001 bis 20 %, bevorzugt 0,01 bis 15 %, besonders bevorzugt 0,1 bis 10 % und insbesondere bevorzugt 0,5 bis 5 % Polyquaternium-91 enthält.

14. Verwendung eines Mittels nach einem der Ansprüche 1 bis 13 zur Verbesserung des Farbaufzugs von Färbemitteln auf keratinische Fasern und/oder zur Verbesserung der Waschstabilität von gefärbten keratinischen Fasern.

15. Verfahren zum Färben und/oder Aufhellen von keratinischen Fasern, **dadurch gekennzeichnet, dass**

- gewünschtenfalls ein Vorbehandlungsmittel M1 auf die Faser aufgebracht wird, dann
- ein Färbe- und/oder Aufhellmittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von 2-25 Minuten wieder abgespült wird,

wobei das Mittel M2 ein erfindungsgemäßes Mittel nach einem der Ansprüche 1 bis 13 ist.

Europäisches Patentamt
European Patent Office
Office européen des brevets

# EUROPÄISCHER RECHERCHENBERICHT

Nummer der Anmeldung
EP 12 18 7854

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DATABASE GNPD [Online] MINTEL; 11. September 2011 (2011-09-11), "Nature'^s Hair Color Permanent Hair Color", XP002717552, Database accession no. 1659074 * Zusammenfassung * ----- | 1-15 | INV. A61Q5/10 A61K8/81 |
| X | "PERSONAL CARE PRODUCT INDEX BY APPLICATION", INTERNET CITATION, 5. September 2006 (2006-09-05), XP002432075, Gefunden im Internet: URL:http://www.interpolymer.com/stuff/contentmgr/files/def701fba57cb2917634527e986f4c7f/miscdocs/PC%20Formulary%205-9-06.pdf [gefunden am 2007-05-03] * Seiten 26, 32 * ----- | 1-15 | |
| A | US 2010/275943 A1 (SALEM ROBERT [US] ET AL) 4. November 2010 (2010-11-04) * das ganze Dokument * ----- | 1-15 | RECHERCHIERTE SACHGEBIETE (IPC) A61K A61Q |
| A | WO 2008/064971 A1 (UNILEVER PLC [GB]; UNILEVER NV [NL]; UNILEVER HINDUSTAN [IN]; ADAMS GE) 5. Juni 2008 (2008-06-05) * das ganze Dokument * ----- | 1-15 | |
| A | WO 2009/085838 A1 (ISP INVESTMENTS INC [US]; YAN ZHOU [US]; DONNA LAURA N [US]; RAYMOND R) 9. Juli 2009 (2009-07-09) * das ganze Dokument * ----- | 1-15 | |

Der vorliegende Recherchenbericht wurde für alle Patentansprüche erstellt

2

| Recherchenort | Abschlußdatum der Recherche | Prüfer |
|---|---|---|
| München | 10. Dezember 2013 | Mitchell, Gemma |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 12 18 7854

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-12-2013

| Im Recherchenbericht angeführtes Patentdokument | Datum der Veröffentlichung | Mitglied(er) der Patentfamilie | Datum der Veröffentlichung |
|---|---|---|---|
| US 2010275943 A1 | 04-11-2010 | KEINE | |
| WO 2008064971 A1 | 05-06-2008 | BR PI0717720 A2 | 22-10-2013 |
| | | EP 2099427 A1 | 16-09-2009 |
| | | JP 2010511002 A | 08-04-2010 |
| | | RU 2009124441 A | 10-01-2011 |
| | | US 2010061954 A1 | 11-03-2010 |
| | | WO 2008064971 A1 | 05-06-2008 |
| WO 2009085838 A1 | 09-07-2009 | EP 2234673 A1 | 06-10-2010 |
| | | US 2011219552 A1 | 15-09-2011 |
| | | WO 2009085838 A1 | 09-07-2009 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 7066966 B
- US 7147672 B
- US 20110219552 A1
- US 20110044924 A


### In der Beschreibung aufgeführte Nicht-Patentliteratur


- Silicones Used in Permanent and Semi-Permanent Hair Dyes to Reduce the Fading and Color Change Process of Dyed Hair Occuring by Wash-Out or UV Radiation. *J. Cosmetic Sci.,* 2004, vol. 55, 123-131
- **KH. SCHRADER.** Grundlagen und Rezepturen der Kosmetika. Hüthig Buch Verlag, 1989